# EUROPÄISCHE PATENTSCHRIFT

(11) **EP 1 885 294 B1**
(45) Veröffentlichungstag und Bekanntmachung des Hinweises auf die Patenterteilung: **19.03.2014**
(21) Anmeldenummer: 06753855.3
(22) Anmeldetag: 24.05.2006
(51) Int. Cl.: A61F 2/36

(54) **SCHENKELHALSPROTHESE**
FEMORAL NECK PROSTHESIS
PROTHESE DU COL DU FEMUR

(30) Priorität: 24.05.2005 EP 05011259
(43) Veröffentlichungstag der Anmeldung: 13.02.2008
(73) Patentinhaber: Zimmer GmbH, 8404 Winterthur (CH); Grappiolo, Guido, 17024 Finale Ligure (IT)
(72) Erfinder: GRAPPIOLO, Guido, I-17024 Finale Ligure (IT); WILLI, Roland, CH-8413 Neftenbach (CH)
(74) Vertreter: Manitz, Finsterwald & Partner GbR
(86) Internationale Anmeldenummer: PCT/EP2006/004976
(87) Internationale Veröffentlichungsnummer: WO 2006/125633

(56) Entgegenhaltungen:
- EP-A- 1 120 096
- WO-A-00/09044
- US-A1- 2003 050 704
- US-A1- 2003 163 202
- US-B1- 6 284 002

## Beschreibung

Die Erfindung betrifft eine Schenkelhalsprothese, umfassend einen zur Anordnung im Inneren des Femurs vorgesehenen Verankerungsbereich zur Verankerung der Prothese im Knochen, einen sich axial an einer proximalen Seite an den Verankerungsbereich anschliessenden Kopfbereich, der eine Vorrichtung zum Verbinden mit einem Prothesenkopf aufweist, sowie ein distales Ende, welches in axialer Richtung des Verankerungsbereichs dem Kopfbereich gegenüberliegt.

Es sind Hüftgelenkprothesen grundsätzlich bekannt, beispielsweise aus WO 00/09044 A1, bei denen sich der Prothesenschaft nicht wie bei schaftfixierten Prothesen in den Femurschaft (Corpus femoris) hinein erstreckt, sondern ohne Verankerung im Femurschaft durch den Femurhals (Collum femoris) verläuft. Derartige Prothesen werden auch als Schenkelhalsprothesen bezeichnet. Bei der in WO 00/09044 A1 beschriebenen Schenkelhalsprothese ist deren Schaft mit lateralem Zugang von distal nach proximal in den Schenkelhals implantierbar, wobei der Schaft eine Möglichkeit zur Verankerung an der inneren Kortikalis des Corpus Femoris bietet. Ein Schaft gemäß dem Oberbegriff von Anspruch 1 ist aus der US-A-2003/0163202 bekannt.

Es soll ein Schaft der eingangs genannten Art angegeben werden, der auf möglichst einfache und sichere Art und Weise am Femur verankert werden kann. Der in Anspruch 1 angegebene Schaft vermag, neben einer Vielzahl weiterer vorteilhafter Eigenschaften, diese Forderung zu erfüllen. Bei dem hier angegebenen Schaft ist vorgesehen, dass die maximale Querschnittsabmessung des Kopfbereichs höchstens gleich groß und insbesondere kleiner ist als ein maximaler Durchmesser des Verankerungsbereichs, derart, dass die maximale Querschnittsdimension des Schaftes im Verankerungsbereich vorliegt. Durch den Verankerungsbereich kann die Verankerung des Schaftes innerhalb eines im Femur ausgebildeten Durchgangs erfolgen. Im Gegensatz zu bekannten Druckscheibenprothesen, bei denen sich die Prothese über ein scheibenförmiges Element an der Resektionsfläche axial abstützt, erfolgt bei dem hier angegebenen Implantat die Fixierung innerhalb einer im Wesentlichen in der Halsachse durch den Femur verlaufenden Bohrung. Insbesondere erweist es sich als günstig, dass dadurch, dass proximal kein Bereich vorliegt, dessen Querschnittsdimension grösser ist als im Verankerungsabschnitt, das Einsetzen der Prothese durch minimal invasive Techniken und insbesondere von lateral erfolgen kann. Da der Verankerungsbereich einen Außenge-windeabschnitt aufweist, können die Gewindeflanken mit einer für einen sicheren Halt ausreichenden und gleichzeitig eine unkritische Beanspruchung der Kortikalis sicherstellenden Höhe versehen sein.

Weitere Ausführungsformen sind in den abhängigen Ansprüchen, der Beschreibung sowie der Zeichnung angegeben, wobei die Merkmale dieser Ausführungsformen in an sich beliebiger Weise miteinander kombinierbar sind.

Der Verankerungsbereich des Schaftes kann einen Zementierabschnitt umfassen und weist erfindungsgemäß einen Außengewindeabschnitt mit einem Spongiosagewinde auf. Diese Ausführung stellt eine Hybridlösung dar, wobei das Gewinde im Wesentlichen zur Aufnahme von Axialkräften und der Zementierabschnitt im Wesentlichen zur Abstützung von Momenten dient. Im Schenkelhalsbereich, wo sich die Kortikalis bzw. relativ kompakte Spongiosa befindet, trägt das Gewinde. Im Übergangsbereich zwischen Trochanter und Schenkelhals liegt eine eher geringe Gewebedichte und Gewebefestigkeit vor. In diesem Bereich stützt sich die Prothese ab, wobei der Knochenzement die eine relativ geringe Dichte aufweisende Spongiosa verfestigt. Das Einbringen des Knochenzements kann durch Einpressen erfolgen. Der Zementierabschnitt kann beispielsweise an seinem Außenumfang wenigstens eine Vertiefung zur Aufnahme von Knochenzement aufweisen. Weiterhin kann der Zementierabschnitt in wenigstens einem axialen Bereich eine im Querschnitt kreuz- oder sternförmige Geometrie aufweisen, wobei im Wesentlichen axial verlaufende Vertiefungen ausgebildet sind. Eine solche Kreuz- oder Sternkonfiguration ist auch für den Einsatz von Instrumenten zum Einschrauben bzw. Manipulieren von lateral geeignet. Die Vertiefungen können sich bis zu dem distalen Ende des Zementierabschnitts erstrecken. Es kann auch vorgesehen sein, dass der Zementierabschnitt wenigstens eine spiral- oder ringförmig umlaufende Nut als Vertiefung zur Aufnahme von Knochenzement aufweist.

Die Vertiefung kann vom distalen Ende des Schaftes aus zugänglich sein. Dies erleichtert bei der Implantation die Handhabung von lateral.

Der Schaft weist in einer beispielhaften Ausführungsform Mittel auf, welche eine Fluidverbindung für die Zufuhr von Knochenzement vom distalen Ende des Schaftes aus zu der Vertiefung gewährleisten, was ebenfalls die Handhabung vereinfacht.

Der Außengewindeabschnitt ist erfindungsgemäß an den Kopfbereich angrenzend angeordnet. Damit kommt dieser in einem Bereich zu liegen, in dem das Gewinde in Spongiosa mit einer vergleichweise hohen Dichte zu liegen kommt und allenfalls mit den Gewindespitzen in der Kortikalis Halt findet.

Das Spongiosagewinde weist beispielsweise einen im Wesentlichen konstanten Kerndurchmesser und/oder einen im Wesentlichen konstanten Außendurchmesser auf. Der maximale Durchmesser des Außengewindes definiert in einer Ausführungsform des beschriebenen Implantats den maximalen Durchmesser des Schaftes. Die Gewindehöhe beträgt dabei in spezifischen Ausführungsformen wenigstens 3 mm und insbesondere wenigstens 4,5 mm.

Des Weiteren kann vorgesehen sein, dass die maximale Querschnittsdimension des Kopfbereichs den maximalen Kerndurchmesser des Gewindes um höchstens 1/3 der Differenz zwischen dem maximalen Außendurchmesser und dem maximalen Kerndurchmesser des Gewindes überschreitet, und insbesondere höchstens gleich groß ist wie der maximale Kerndurchmesser des Gewindes. Dies ermöglicht es, dass der Schaft über eine zur Aufnahme des Verankerungsbereiches vorgesehene Öffnung von lateral eingesetzt werden kann.

Wenn der Schaft einen Zementierabschnitt aufweist, dann kann sich dieser beispielsweise distal an den Gewindeabschnitt anschließen und so eine gute zementunterstützte Abstützung in der Spongiosa ermöglichen.

Die maximale Querschnittsdimension des Zementierabschnitts übersteigt in einer Ausführungsform den maximalen Kerndurchmesser des Gewindes um höchstens 1/3 der Differenz zwischen dem maximalen Außendurchmesser und dem maximalen Kerndurchmesser des Gewindes, und ist insbesondere höchstens gleich groß wie der maximale Kerndurchmesser des Gewindes. Ferner kann vorgesehen sein, dass die maximale Querschnittsdimension des Zementierabschnitts wenigstens so groß ist wie die maximale Querschnittsdimension des Kopfbereichs. Diese optionalen Merkmale gewährleisten einen guten Sitz des Zementierabschnittes in der zum Einsetzen des Implantats hergestellten Bohrung.

Der Zementierabschnitt kann im Bereich seines distalen Endes mit einem Dichtungsabschnitt versehen sein, wobei der Dichtungsabschnitt beispielsweise gewindeförmig ausgebildet ist. Wenn der Verankerungsbereich einen Außengewindeabschnitt mit einem Spongiosagewinde umfasst, dann kann der gewindeförmige Dichtungsabschnitt, insbesondere hinsichtlich der Abmessung des Außendurchmessers und/oder der Gewindehöhe, dem Spongiosagewinde entsprechen.

Der Kopfbereich und der Verankerungsbereich sind beispielsweise koaxial angeordnet. Eine koaxiale Anordnung kann ebenfalls zwischen wenigstens zwei der Bereiche bzw. Abschnitte Kopfbereich, Zementierabschnitt und Außengewindeabschnitt gegeben sein.

Die proximal-distale Erstreckung des Verankerungsbereichs kann derart bemessen sein, dass das distale Ende des Schaftes im Spongiosagewebe des Femurs zu liegen kommt. Der Schaft ragt dann nicht lateral aus dem Femur hervor.

Der Kopfbereich weist beispielsweise ein konisches oder kegelstumpfförmiges Verbindungselement zur Verbindung mit einem Prothesenkopf auf.

Es wird hier auch ein Instrumentensatz angegeben, mit dem der Schaft auf einfache und sichere Art und Weise am Femur verankert und somit die den Schaft umfassende Schenkelhalsprothese ebenfalls auf einfache und sichere Art und Weise eingesetzt werden kann. Der Instrumentensatz umfasst einen Kernlochbohrer und einen Gewindeschneider zum Herstellen eines Durchgangs sowie ein Eindrehinstrument zum Eindrehen des Schaftes in den Durchgang von lateral. Es kann zusätzlich ein Einbringinstrument zum Einbringen von Knochenzement vorgesehen sein, das einen auf das distale Ende des Schaftes aufsetzbaren Einbringabschnitt aufweist. Der Einbringabschnitt kann nach Art einer Tülle ausgebildet sein und dient insbesondere als Adapter zur optimalen Anpassung des Ausströmendes einer herkömmlichen Zementspritze oder -pistole an das distale Ende des Prothesenschaftes. Der Einbringabschnitt kann derart auf das distale Ende des Schaftes abgestimmt sein, dass im aufgesetzten Zustand ein im Schaft ausgebildeter Kanal gegenüber der Umgebung abgedichtet ist. Insbesondere kann der Einbringabschnitt zumindest bereichsweise kappen-, hauben- oder glockenförmig ausgebildet sein. Es kann sich bei dem Einbringabschnitt um ein separates, mit einer Zementspritze oder -pistole verbindbares Bauteil handeln.

Der Instrumentensatz kann bei einem Verfahren zum Einsetzen einer Schenkelhalsprothese der vorstehend beschriebenen Art eingesetzt werden, bei dem über einen vorderen Zugang und entsprechenden Schnitt der Femurkopf entfernt, von lateral ein durch den Femurhals verlaufendes Kernloch ausgebildet, in das Kernloch ein Gewinde geschnitten, in den dadurch gebildeten Durchgang der Prothesenschaft eingedreht und über den vorderen Zugang der Prothesenkopf auf den Schaft aufgesetzt wird.

Der Schaft kann, sofern ein Zementierabschnitt vorgesehen ist, beispielsweise einzementiert werden. Insbesondere wird der Knochenzement bei bereits im Durchgang befindlichem Schaft eingebracht. Zum Einzementieren des Schaftes kann auf dessen distales Ende ein insbesondere tüllenartig ausgebildeter Einbringabschnitt eines Einbringinstruments aufgesetzt werden.

Die Bearbeitung des Acetabulum (Hüftgelenkspfanne) des Hüftknochens zum Einsetzen der Prothesenschale erfolgt insbesondere über den vorderen Zugang (anterior), über den vorher der Femurkopf entfernt wird. Alternativ ist es auch möglich, diese Bearbeitung über das zuvor ausgebildete Kernloch bzw. den fertigen Durchgang durchzuführen. Hierbei kann in das Kernloch bzw. den fertigen Durchgang eine Hülse eingeschoben werden, durch welche anschließend Spindeln für die notwendigen Bearbeitungsinstrumente geführt und die eigentlichen Instrumente auf die durchgesteckten Spindeln aufgesteckt werden. Diese Hülse kann zur Fixierung des Femur genutzt werden, indem das Femur über einen nach außen vorstehenden Abschnitt der Hülse in einer korrekt relativ zum Hüftknochen ausgerichteten Stellung insbesondere am Operationstisch fixiert wird. Die Prothesenschale kann über den vorderen Zugang, über den der Femurkopf entfernt wird, eingesetzt werden, wobei dies nach dem Ausbilden des Kernlochs und vor dem Schneiden des Gewindes erfolgen kann.

Eine Verankerung des Prothesenschaftes mittels eines im Durchgang ausgebildeten Gewindes ermöglicht es, dass eine proximal-distale Lagekorrektur durch Verdrehen des Schaftes im Durchgang vorgenommen werden kann, wobei dies nach dem Aufsetzen des Prothesenkopfes erfolgen kann.

Alle sowohl in den Ansprüchen als auch in der Beschreibung und in den Zeichnungen in Verbindung mit der Prothese gegebenenfalls verwendeten und gemäß der fachüblichen Konvention gewählten Ausrichtungs-, Positionierungs-, Orientierungs- und Richtungsangaben, die insbesondere anatomische Achsen, Ebenen, Richtungen im Raum und Bewegungsrichtungen betreffen, sind dem Fachmann bekannt und beziehen sich auf den implantierten Zustand der Prothese.

Die Erfindung wird im Folgenden beispielhaft unter Bezugnahme auf die Zeichnung beschrieben. Es zeigen:
- Fig. 1a: verschiedene Ansichten eines Prothesenschaftes einer Hüftgelenkprothese gemäß einer ersten Ausführungsform,
- Fig. 1b: Ansichten entsprechend Fig. 1a eines Prothesenschaftes gemäß einer zweiten Ausführungsform,
- Fig. 2: einen Gewindeschneider eines Instrumentensatzes,
- Fig. 3: das Eindrehen des Prothesenschaftes der ersten Ausführungsform mit einem Eindrehinstrument,
- Fig. 4: das Einbringen von Knochenzement bei einem Prothesenschaft der ersten Ausführungsform mit einem Einbringinstrument,
- Fig. 5: einen Prothesenschaft der ersten Ausführungsform im eingesetzten Zustand mit aufgesetztem Prothesenkopf,
- Fig. 6: verschiedene Ansichten eines Prothesenschaftes einer Hüftgelenkprothese gemäß einer dritten Ausführungsform,
- Fig. 7: das Eindrehen des Prothesenschaftes der dritten Ausführungsform mit einem anderen Eindrehinstrument,
- Fig. 8: das Einbringen von Knochenzement bei einem Prothesenschaft der dritten Ausführungsform mit einem anderen Einbringinstrument, und
- Fig. 9: einen Prothesenschaft der dritten Ausführungsform im eingesetzten Zustand mit aufgesetztem Prothesenkopf.

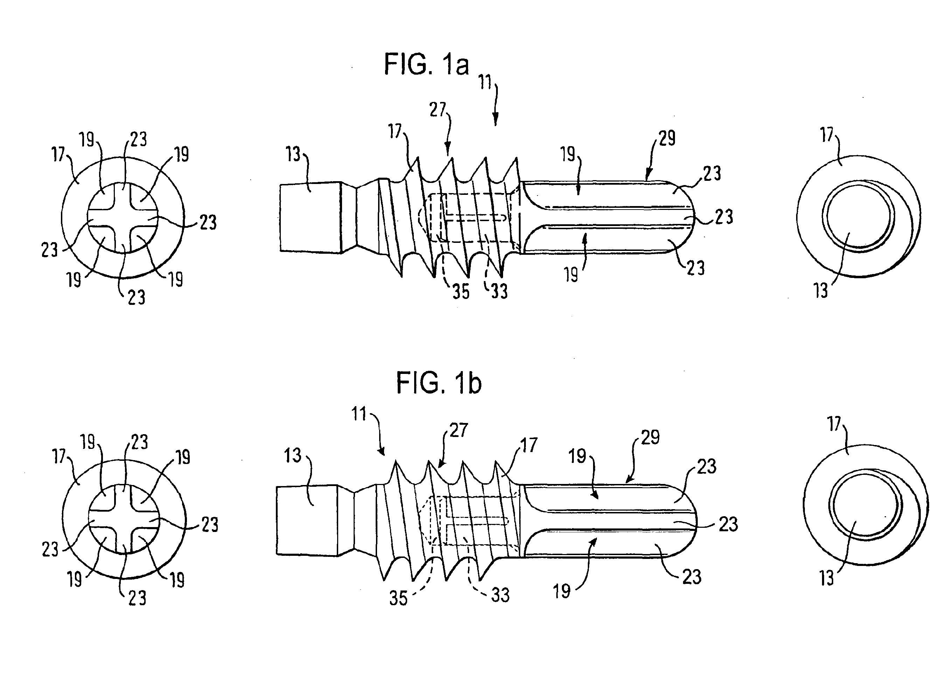

Von der Hüftgelenkprothese gemäß der ersten Ausführungsform ist in Fig. 1a lediglich der Prothesenschaft 11 dargestellt. Der Schaft 11 ist mehrteilig ausgeführt und umfasst einen distalen Zementierabschnitt 29 und einen auch als Kopfbereich bezeichneten proximalen Abschnitt, der am proximalen Ende als Konus 13 ausgebildet ist. Ein Verankerungsbereich umfasst den Zementierabschnitt 29 sowie einen sich nach distal an den Konus 13 anschließenden, mit einem als Spongiosagewinde ausgebildeten Außengewinde 17 versehenen Abschnitt 27. Der Konus 13 dient zur Kopplung mit einem hier nicht dargestellten, eine entspre-chend geformte Aufnahme für den Konus 13 aufweisenden Prothesenkopf. Ferner umfasst die Prothese eine hier ebenfalls nicht dargestellte Gelenkschale zum Einsetzen in den Hüftknochen. Zur Bildung des Schaftes 11 sind die beiden beschriebenen Schaftteile 27, 29 fest zusammengesteckt. Hierzu ist der Zementierabschnitt 29 mit einem Verbindungszapfen 33 versehen, für den im Gewindeabschnitt 27 eine entsprechende Zapfenaufnahme 35 ausgebildet ist. Der Zementierabschnitt 29 besitzt einen sternförmigen Querschnitt, der durch vier jeweils paarweise rechtwinklig zueinander stehende, sich in axialer Richtung erstreckende Stege 23 gebildet ist, die in axialer Richtung lang gestreckte, nach distal offene Vertiefungen 19 zur Aufnahme von Knochenzement in Umfangsrichtung begrenzen.

Fig. 1b zeigt eine zweite Ausführungsform, die sich von der ersten Ausführungsform (Fig. 1 a) durch die Ausgestaltung des Spongiosagewindes 17 unterscheidet. Bezogen auf die Axialrichtung ist die Anordnung der Gewindeflanken bei der zweiten Ausführungsform relativ zur ersten Ausführungsform umgekehrt.

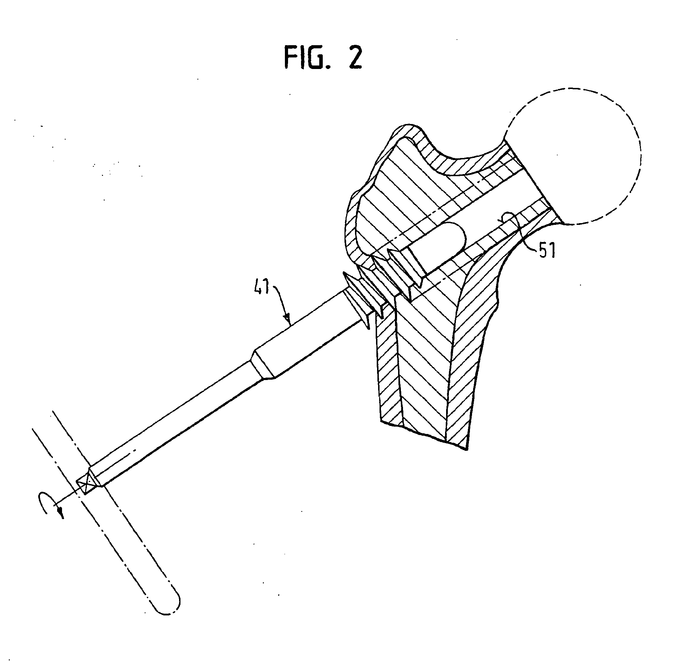

Fig. 2 zeigt, wie am Femur von lateral kommend in Verlängerung der Femurhalsachse mittels eines Gewindeschneiders 41 ein Gewinde in ein zuvor ausgebildetes Kernloch 51 geschnitten wird. Der Femurkopf ist lediglich gestrichelt dargestellt, da er in dieser Phase des Operationsverfahrens, auf das nachstehend näher eingegangen wird, bereits entfernt ist.

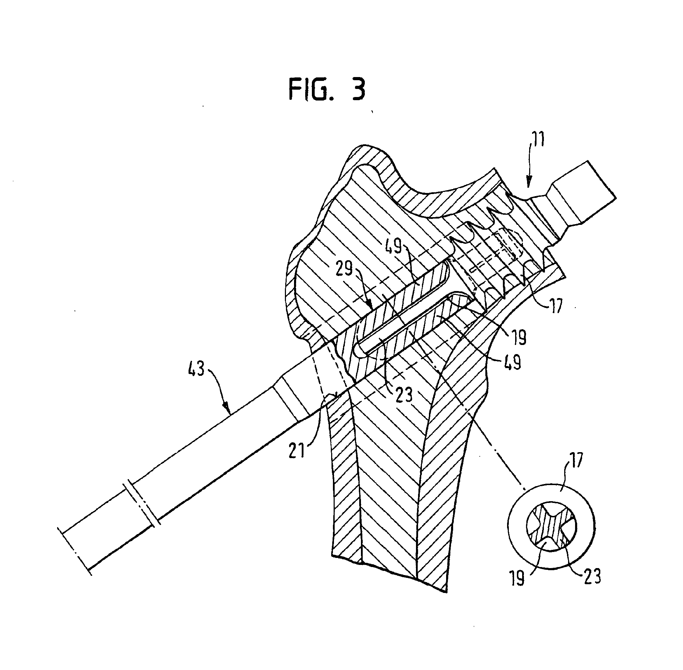

Gemäß Fig. 3 umfasst der Instrumentensatz außerdem ein Eindrehinstrument 43, das auf den distalen Zementierabschnitt 29 des Prothesenschaftes 11 abgestimmt ist. Das Eindrehinstrument 43 ist mit Fingern 49 versehen, die in die Vertiefungen 19 des Zementierabschnitts 29 eingreifen. Das Eindrehinstrument 43 ist insofern komplementär zu dem Zementierabschnitt 29 des Schaftes 11 ausgebildet.

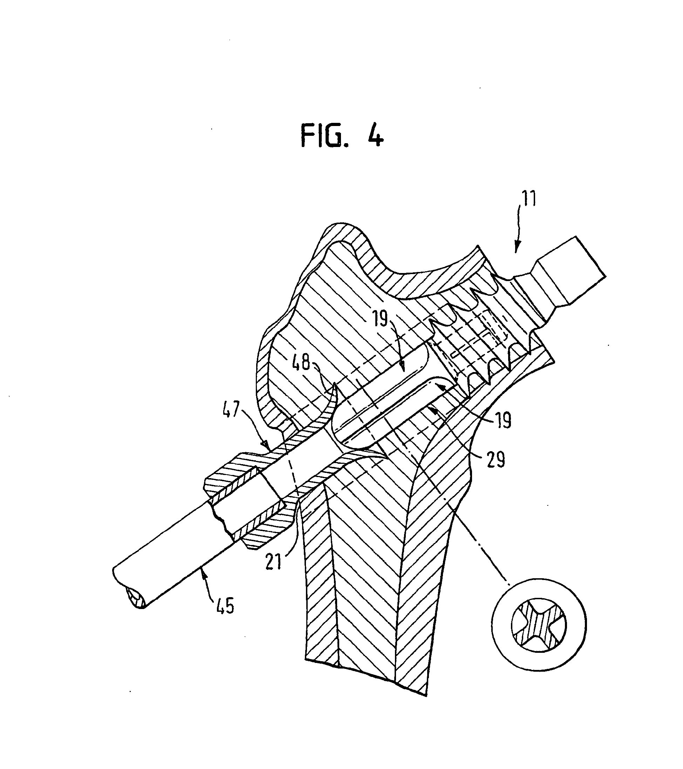

Zum Einzementieren des Prothesenschaftes 11 dient gemäß Fig. 4 ein Einbringinstrument 45 des Instrumentensatzes, das einen an die Formgebung des hinteren Endes des Zementierabschnitts 29 angepassten Einbringabschnitt 47 umfasst. Der Einbringabschnitt 47 ist als separates Bauteil vorgesehen, das auf eine herkömmliche Zementspritze oder -pistole aufgesteckt werden kann und somit als eine Art Adaptertülle dient. Der Austrittsbereich des Einbringabschnitts 47 ist als glockenförmiger Kontaktbereich 48 ausgebildet, der radial über den lediglich den Kernlochdurchmesser aufweisenden Zementierabschnitt 29 vorsteht und durch seine Formgebung eine radiale Ausbreitung des eingespritzten Knochenzements unterstützt sowie ein Austreten von Knochenzement aus dem Durchgang 21 verhindert. Der eingespritzte Knochenzement gelangt über den Einbringabschnitt 48 in die Vertiefungen 19 des Zementierabschnitts 29 und von dort in im Wesentlichen radialer Richtung in das offenporige Material der Spongiosa des Femurknochens.

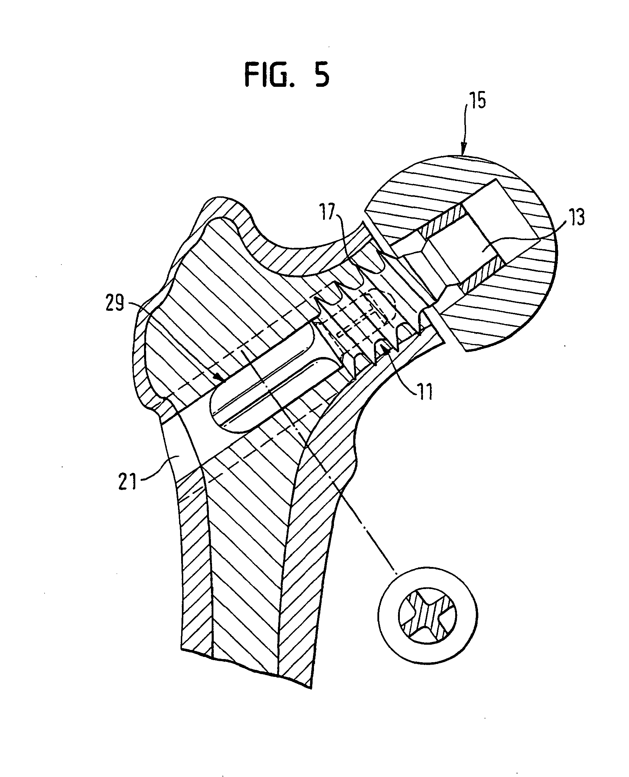

Im komplettierten Zustand gemäß Fig. 5 befindet sich der mit dem auf den Konus 13 aufgesetzten Prothesenkopf 15 versehene Prothesenschaft 11 in der korrekten Tiefe innerhalb des Durchgangs 21 und ist im relativ harten Bereich des Femurhalses über das proximale Gewinde 17 und in der relativ offenporigen Spongiosa über den Zementierabschnitt 29 am Femur verankert. Es wird somit der Prothesenschaft 11 praktisch über seine gesamte axiale Länge im Femur fixiert, ohne dass externe Druck- oder Spanneinrichtungen erforderlich wären, um den Prothesenschaft 11 zu befestigen.

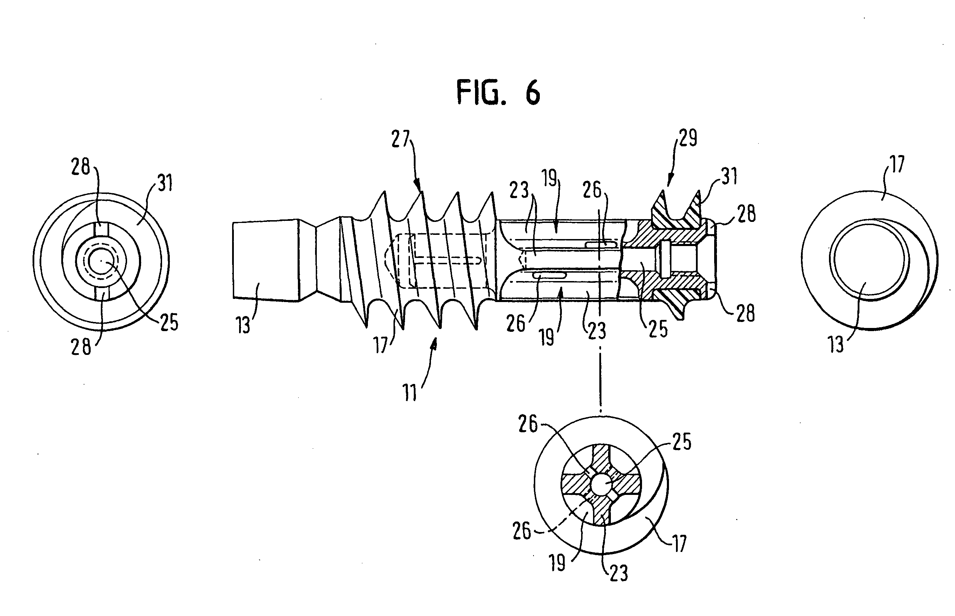

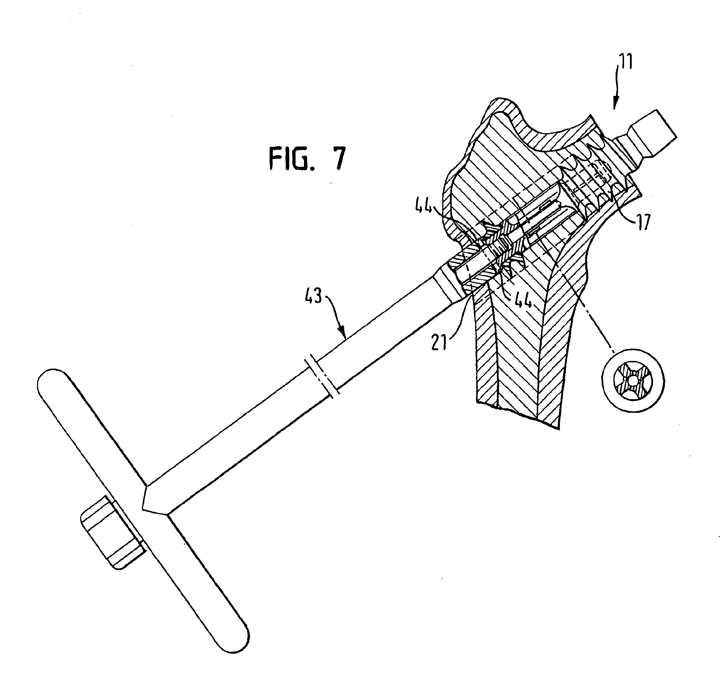

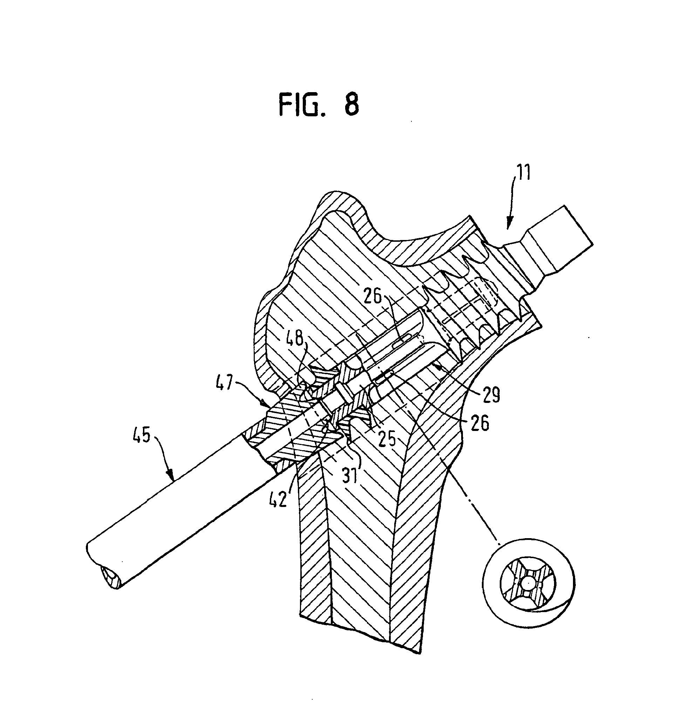

Die in Fig. 6 dargestellte dritte Ausführungsform eines Prothesenschaftes 11 unterscheidet sich von den beiden zuvor beschriebenen Ausführungsformen durch die Ausgestaltung des Zementierabschnitts 29. Die wiederum vorgesehenen, sich in axialer Richtung zwischen stegartigen Wandabschnitten 23 erstreckenden Vertiefungen 19 sind hier nicht nach distal offen und werden nicht direkt mit Knochenzement beschickt. Vielmehr ist der Zementierabschnitt 29 mit einem axial verlaufenden, zentralen Einbringkanal 25 versehen, der von distal zugänglich ist und mit den Vertiefungen 19 über radiale Durchbrüche 26 kommuniziert. Diese Öffnungen 26 sind dabei bezüglich der axialen Richtung zueinander versetzt angeordnet. Im Bereich seines distalen Endes ist der Zementierabschnitt 29 mit einem Gewindeabschnitt 31 versehen, der beim Einbringen des Knochenzements als Dichtung dient, worauf nachstehend näher eingegangen wird. Der Gewindeabschnitt 31 ist auf das proximale Verankerungsgewinde 17 abgestimmt und insbesondere als dessen Fortsetzung ausgelegt. Da der primäre Zweck des Gewindeabschnitts 31 nicht die Verankerung des Schaftes 11 im Femurknochen ist, kann der Gewindeabschnitt 31 z.B. aus einem bioresorbierbaren Material hergestellt sein. Die distale Stirnseite des Zementierabschnitts 29 ist mit zwei einander diametral gegenüberliegenden Aussparungen 28 versehen. Wie Fig. 7 zeigt, dienen diese Aussparungen 28 zur Aufnahme von entsprechend geformten Vorsprüngen 44 eines Eindrehinstruments 43, mit dem der Schaft 11 in den zuvor hergestellten Durchgang 21 im Femurknochen eingedreht wird. Nicht nur dieses Eindrehinstrument 43, sondern auch das Instrument 45 zum Einbringen von Knochenzement unterscheidet sich von dem entsprechenden Instrument für die erste und zweite Ausführungsform. Wie Fig. 8 zeigt, weist der das distale Ende des Zementierabschnitts 29 im aufgesetzten Zustand abdichtende Kontaktbereich 48 der Tülle 47 zusätzlich zu einer radial äußeren Ringwand, die das distale Ende des Zementierabschnitts 29 außen umfasst, eine radial innere Ringwand 42, die den Kanal 25 des Zementierabschnitts 29 nach außen abdichtet. Anders als beim Einbringinstrument für die erste und zweite Ausführungsform des Prothesenschaftes hat bei dem in Fig. 8 dargestellten Einbringinstrument 45 die radial äußere Ringwand des Kontaktbereiches 48 keine Lenkungs- und Abdichtfunktion. Der axial verlaufende Kanal 25 und die in die Vertiefungen 19 mündenden Durchbrüche 26 sorgen für ein radiales Ausströmen des Knochenzementes aus den Vertiefungen 19. Die Abdichtung des Durchgangs 21 ist durch die als Gewinde ausgebildete Dichtung 31 gewährleistet.

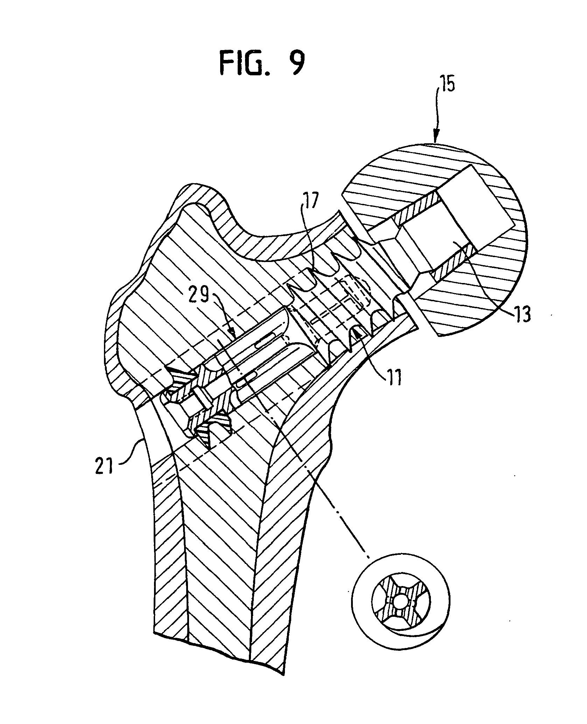

Somit ist auch der Prothesenschaft 11 der dritten Ausführungsform gemäß Fig. 9 über das Gewinde 17 im vergleichsweise harten Material des Femurhalses und über den mit dem Zementierabschnitt 29 zusammenwirkenden Knochenzement im vergleichsweise offenporigen Material der Spongiosa am Femurknochen sicher verankert.

Unabhängig davon, welcher Zementierabschnitt 29 für den Prothesenschaft 11 zum Einsatz kommt, ist der Ablauf des Operationsverfahrens zum Einsetzen der Prothese wie folgt:
Zunächst wird über einen vorderen Zugang (anterior) der Femurkopf abgetrennt und herausgenommen. Anschließend wird entweder gleich das Acetabulum des Hüftknochens bearbeitet, und zwar ebenfalls durch den erwähnten vorderen Zugang, oder es wird zunächst die ohnehin benötigte Kernlochbohrung 51 (vgl. z.B. Fig. 2) ausgebildet. Dies erfolgt mit Hilfe eines computergestützten optischen Navigationssystems. Zur Bearbeitung des Acetabulums über die Kernlochbohrung 51 wird in diese eine nicht dargestellte Bearbeitungshülse eingeschoben, über welche anschließend die für die Bearbeitung des Acetabulums benötigten Instrumente zugeführt werden können. Die hierbei erforderliche korrekte Ausrichtung des Femurknochens relativ zum Hüftknochen kann dadurch fixiert werden, dass die Bearbeitungshülse derart bemessen wird, dass sie lateral aus dem Oberschenkel des Patienten herausragt und am Operationstisch durch geeignete Mittel festgehalten wird. Das anschließende Einsetzen der nicht dargestellten Prothesenschale erfolgt wiederum über den erwähnten vorderen Zugang. Dann wird nach Entnahme der Bearbeitungshülse mittels des Gewindeschneiders in die Kernlochbohrung 51 ein durchgehendes Innengewinde für den Prothesenschaft 11 ausgebildet. Anschließend wird mittels des Eindrehinstruments 43 (vgl. Fig. 3 bzw. Fig. 7) der Prothesenschaft 11 in eine zuvor im Rahmen der Operationsplanung festgelegte Tiefe eingedreht. Daraufhin wird - wiederum über den erwähnten vorderen Zugang - der Prothesenkopf 15 eingesetzt und auf den Konus 15 des Prothesenschaftes 11 aufgesetzt. Auch die zur Fixierung des Kopfes 15 auf dem Konus 13 erforderlichen Schläge auf den Kopf 15 werden durch den vorderen Zugang mittels entsprechender, hier nicht dargestellter Instrumente vorgenommen. Anschließend kann die korrekte Lage des Prothesenkopfes 15 kontrolliert und erforderlichenfalls durch Verdrehen des Prothesenschaftes 11 im Durchgang 21 korrigiert werden. Ist die korrekte Lage, d.h. die korrekte Tiefe des Prothesenschaftes 11 im Durchgang 21, erreicht, wird der Prothesenschaft 11 mit Hilfe des Einbringinstruments 45 (vgl. Fig. 4 bzw. Fig. 8) einzementiert. Hierzu wird die jeweilige "Tülle" 47, die an einer herkömmlichen Zementspritze angebracht ist, auf das distale Ende des Prothesenschaftes 11 gesetzt, wie es vorstehend erläutert wurde. Sobald der Zement ausgehärtet ist, ist der Prothesenschaft 11 nicht nur über sein Gewinde 17, sondern außerdem über den Zementierabschnitt 29 sicher im Femurknochen verankert.

Eine Eigenschaft der hier angegebenen Prothese besteht darin, dass mit ausschließlich minimal invasiven Operationstechniken gearbeitet werden kann und dass ein Einsetzen des Schaftes von lateral erfolgen kann, wobei lediglich das Aufsetzen des Prothesenkopfes von medial erfolgen muss.

### Bezugszeichenliste

- 11: Schaft der Prothese
- 13: proximaler Endbereich, Kopplungsabschnitt, Konus
- 15: Kopf der Prothese
- 17: Gewinde des Prothesenschaftes, Spongiosagewinde
- 19: Vertiefung, Aufnahme
- 21: Durchgang
- 23: Wandabschnitt, Steg
- 25: Kanal
- 26: Öffnung, Durchbruch
- 27: Verankerungsabschnitt mit Gewinde
- 28: Aussparung für Eindrehinstrument
- 29: Verankerungsabschnitt mit Vertiefung, Zementierabschnitt
- 31: Dichtungsabschnitt, hinterer Gewindeabschnitt
- 33: Verbindungszapfen
- 35: Zapfenaufnahme
- 41: Gewindeschneider
- 42: radial innere Ringwand
- 43: Eindrehinstrument
- 44: Vorsprung des Eindrehinstruments
- 45: Einbringinstrument, Zementspritze bzw. -pistole
- 47: Einbringabschnitt, Tülle
- 48: Kontaktbereich
- 49: Finger des Eindrehinstruments
- 51: Kernlochbohrung

## Patentansprüche

1. Schaft für eine Schenkelhalsprothese,
umfassend einen zur Anordnung im Inneren des Femurs vorgesehenen Verankerungsbereich (27, 29) zur Verankerung der Prothese im Knochen, einen sich axial an einer proximalen Seite an den Verankerungsbereich (27, 29) anschliessenden Kopfbereich, der eine Vorrichtung (13) zum Verbinden mit einem Prothesenkopf (15) aufweist, sowie ein distales Ende, welches in axialer Richtung des Verankerungsbereichs (27, 29) dem Kopfbereich gegenüberliegt, wobei
die maximale Querschnittsabmessung des Kopfbereichs höchstens gleich groß, insbesondere kleiner ist, als ein maximaler Durchmesser des Verankerungsbereichs (27, 29), derart, dass die maximale Querschnittsdimension des Schaftes (11) im Verankerungsbereich (27, 29) vorliegt, **dadurch gekennzeichnet,**
**dass** der Verankerungsbereich einen einstückig mit dem Kopfbereich ausgebildeten Außengewindeabschnitt (27) mit einem Spongiosagewinde (17) umfasst, wobei der Außengewindeabschnitt (27) an den Kopfbereich angrenzend angeordnet ist.

2. Schaft nach Anspruch 1,
**dadurch gekennzeichnet ,**
**dass** der Verankerungsbereich einen Zementierabschnitt (29) umfasst.

3. Schaft nach Anspruch 2,
**dadurch gekennzeichnet,**
**dass** der Zementierabschnitt (29) an seinem Außenumfang wenigstens eine Vertiefung (19) zur Aufnahme von Knochenzement aufweist.

4. Schaft nach Anspruch 3,
**dadurch gekennzeichnet,**
**dass** die Vertiefung (19) vom distalen Ende des Schaftes (11) aus zugänglich ist.

5. Schaft nach einem Ansprüche 3 bis 4,
**dadurch gekennzeichnet,**
**dass** der Schaft (11) Mittel (25, 26) aufweist, welche eine Fluidverbindung für die Zufuhr von Knochenzement vom distalen Ende des Schaftes (11) aus zu der Vertiefung (19) gewährleisten.

6. Schaft nach einem der vorhergehenden Ansprüche,
**dadurch gekennzeichnet ,**
**dass** der maximale Durchmesser des Außengewindes (17) den maximalen Durchmesser des Schaftes (11) definiert.

7. Schaft nach einem der vorhergehenden Ansprüche,
**dadurch gekennzeichnet ,**
**dass** der Schaft (11) einen Zementierabschnitt (29) aufweist, wobei vorzugsweise sich der Zementierabschnitt (29) distal an den Gewindeabschnitt (27) anschließt.

8. Schaft nach Anspruch 7,
**dadurch gekennzeichnet ,**
**dass** die maximale Querschnittsdimension des Zementierabschnitts (29) wenigstens so groß ist wie die maximale Querschnittsdimension des Kopfbereichs.

9. Schaft nach einem der vorstehenden Ansprüche,
**dadurch gekennzeichnet,**
**dass** der Verankerungsbereich einen Zementierabschnitt (29) und einen Außengewindeabschnitt (27) umfasst, wobei der Kopfbereich und der Zementierabschnitt (29) und/oder der Außengewindeabschnitt (27) koaxial angeordnet sind, oder wobei der Zementierabschnitt (29) und der Außengewindeabschnitt (27) koaxial angeordnet sind.

10. Schaft nach einem der vorstehenden Ansprüche,
**dadurch gekennzeichnet ,**
**dass** die proximal-distale Erstreckung des Verankerungsbereichs (27, 29) derart bemessen ist, dass das distale Ende des Schaftes (11) im Spongiosagewebe des Femurs zu liegen kommt.

## Claims

1. A shaft for a femoral neck prosthesis,
comprising an anchorage region (27, 29) provided for arrangement in the interior of the femur for the anchorage of the prosthesis in the bone, a head region which adjoins the anchorage region (27, 29) axially at a proximal side and which has an apparatus (13) for connection to a prosthesis head (15), and a distal end which is disposed opposite the head region in the axial direction of the anchorage region (27, 29), wherein
the maximum cross-sectional dimension of the head region is at most the same size as, in particular smaller than, a maximum diameter of the anchorage region (27, 29) such that the maximum cross-sectional dimension of the shaft (11) is present in the anchorage region (27, 29),
**characterized in that**
the anchorage region comprises an external thread section (27) which is formed in one piece with the head region and which has a spongiosa thread (17), with the external thread section (27) being arranged adjacent to the head region.

2. A shaft in accordance with claim 1,
**characterized in that**
the anchorage region comprises a cementing section (29).

3. A shaft in accordance with claim 2,
**characterized in that**
the cementing section (29) has at least one recess (19) for the reception of bone cement at its outer periphery.

4. A shaft in accordance with claim 3,
**characterized in that**
the recess (19) is accessible from the distal end of the shaft (11).

5. A shaft in accordance with one of the claims 3 to 4,
**characterized in that**
the shaft (11) has means (25, 26) which ensure a fluid connection for the supply of bone cement from the distal end of the shaft (11) to the recess (19).

6. A shaft in accordance with any one of the preceding claims,
**characterized in that**
the maximum diameter of the external thread (17) defines the maximum diameter of the shaft (11).

7. A shaft in accordance with any one of the preceding claims,
**characterized in that**
the shaft (11) has a cementing section (29), with the cementing section (29) preferably adjoining the threaded section (27) at distal.

8. A shaft in accordance with claim 7,
**characterized in that**
the maximum cross-sectional dimension of the cementing section (29) is at least the same size as the maximum cross-sectional dimension of the head region.

9. A shaft in accordance with any one of the preceding claims,
**characterized in that**
the anchorage region comprises a cementing region (29) and an external thread section (27), with the head region and the cementing section (29) and/or the external thread section (27) being arranged coaxially, or with the cementing section (29) and the external thread section (27) being arranged coaxially.

10. A shaft in accordance with any one of the preceding claims,
**characterized in that**
the proximal to distal extent of the anchorage region (27, 29) is dimensioned such that the distal end of the shaft (11) comes to lie in the spongiosa tissue of the femur.

## Revendications

1. Tige pour une prothèse du col du fémur,
comprenant une zone d'ancrage (27, 29), prévue pour être agencée à l'intérieur du fémur, pour l'ancrage de la prothèse dans l'os, une zone de tête qui se raccorde axialement sur un côté proximal à la zone d'ancrage (27, 29) et qui comporte un dispositif (13) pour la liaison avec une tête de prothèse (15), ainsi qu'une extrémité distale qui est à l'opposé de la zone de tête en direction axiale de la zone d'ancrage (27, 29), dans laquelle la dimension de section maximale de la zone de tête est au maximum égale et en particulier inférieure à un diamètre maximum de la zone d'ancrage (27, 29), de telle façon que la dimension de section maximum de la tige (11) se trouve dans la zone d'ancrage (27, 29), **caractérisée en ce que** la zone d'ancrage comprend une portion avec filetage extérieur (27) réalisée d'une seule pièce avec la zone de tête et dotée d'un filetage destiné au corps spongieux (17), et la portion avec filetage extérieur (27) est agencée adjacente à la zone de tête.

2. Tige selon la revendication 1,
**caractérisée en ce que** la zone d'ancrage comprend une portion à cimenter (29).

3. Tige selon la revendication 2,
**caractérisée en ce que** la portion à cimenter (29) comporte à sa périphérie extérieure au moins un renfoncement (19) pour recevoir du ciment osseux.

4. Tige selon la revendication 3,
**caractérisée en ce que** le renfoncement (19) est accessible depuis l'extrémité distale de la tige (11).

5. Tige selon l'une des revendications 3 à 4,
**caractérisée en ce que** la tige (11) comprend des moyens (25, 26) qui assurent une liaison fluidique pour l'alimentation de ciment osseux depuis l'extrémité distale de la tige (11) jusqu'au renfoncement (19).

6. Tige selon l'une des revendications précédentes,
**caractérisée en ce que** le diamètre maximum du filetage extérieur (17) définit le diamètre maximum de la tige (11).

7. Tige selon l'une des revendications précédentes,
**caractérisée en ce que** la tige (11) comprend une portion à cimenter (29), et de préférence la portion à cimenter (19) se raccorde en direction distale à la portion filetée (27).

8. Tige selon la revendication 7,
**caractérisée en ce que** la dimension de section maximum de la portion à cimenter (29) est au moins aussi grande que la dimension de section maximum de la zone de tête.

9. Tige selon l'une des revendications précédentes,
**caractérisée en ce que** la zone d'ancrage comprend une portion à cimenter (29) et une portion avec filetage extérieur (27), dans laquelle la zone de tête et la portion à cimenter (29) et/ou la portion avec filetage extérieur (27) sont agencées coaxialement, ou dans laquelle la portion à cimenter (29) et la portion avec filetage extérieur (27) sont agencées coaxialement.

10. Tige selon l'une des revendications précédentes,
**caractérisée en ce que** l'extension proximale-distale de la zone d'ancrage (27, 29) est choisie de telle façon que l'extrémité distale de la tige (11) vient se placer dans le tissu spongieux du fémur.
